# EUROPÄISCHE PATENTSCHRIFT

(11) **EP 2 222 292 B1**
(45) Veröffentlichungstag und Bekanntmachung des Hinweises auf die Patenterteilung: **04.06.2014**
(21) Anmeldenummer: 08852686.8
(22) Anmeldetag: 18.11.2008
(51) Int. Cl.: A61K 31/202, A61P 29/00, A61K 45/06

(54) **NEUE VERWENDUNG VON OMEGA-3-FETTSÄURE(N)**
NOVEL USE OF OMEGA-3-FATTY ACID(S)
NOUVELLE UTILISATION D'ACIDE(S) GRAS OMÉGA-3

(30) Priorität: 19.11.2007 DE 102007055344
(43) Veröffentlichungstag der Anmeldung: 01.09.2010
(73) Patentinhaber: K.D. Pharma Bexbach GMBH, 66450 Bexbach (DE)
(72) Erfinder: KRUMBHOLZ, Rudolf, 66589 Merchweiler (DE); KRUMBHOLZ, Karin, 66589 Merchweiler (DE); KRUMBHOLZ, Christine, 66589 Merchweiler (DE); SCHIRRA, Norbert, 66333 Völklingen (DE); LEMBKE, Peter, 66453 Gersheim-Reinheim (DE)
(74) Vertreter: Wolff, Matthias
(86) Internationale Anmeldenummer: PCT/DE2008/001928
(87) Internationale Veröffentlichungsnummer: WO 2009/065395

(56) Entgegenhaltungen:
- WO-A-00/51576
- WO-A-98/52556
- WO-A-02/096408
- WO-A-2004/004638
- WO-A-2008/088808
- WO-A2-03/043570
- DE-A1-102005 005 086
- GB-A- 2 409 644
- US-A- 5 709 855
- US-A1- 2001 044 410
- US-A1- 2005 192 352
- US-A1- 2005 226 945
- US-A1- 2005 249 823
- US-A1- 2006 233 892
- SIMOPOULOS ARTEMIS P: "Omega-3 fatty acids in inflammation and autoimmune diseases.", JOURNAL OF THE AMERICAN COLLEGE OF NUTRITION DEC 2002 LNKD- PUBMED:12480795, vol. 21, no. 6, December 2002 (2002-12), pages 495-505, ISSN: 0731-5724
- LENN JON ET AL: "The effects of fish oil and isoflavones on delayed onset muscle soreness", MEDICINE AND SCIENCE IN SPORTS AND EXERCISE, LIPPINCOTT WILLIAMS & WILKINS, BALTIMORE, MD, US, vol. 34, no. 10, 1 October 2002 (2002-10-01), pages 1605-1613, XP008142601, ISSN: 0195-9131
- Mutschler, Ernst: "Arzneimittelwirkungen", Wissenschaftliche Verlagsgesellschaft mbH Stuttgart pages 1998-1999,
- MAROON J C ET AL: "omega-3 Fatty acids (fish oil) as an anti-inflammatory: an alternative to nonsteroidal anti-inflammatory drugs for discogenic pain", SURGICAL NEUROLOGY, LITTLE, BROWN AND CO., BOSTON, MA, US, vol. 65, no. 4, 1 April 2006 (2006-04-01), pages 326-331, XP027927688, ISSN: 0090-3019 [retrieved on 2006-04-01]

## Beschreibung

Die Erfindung betrifft eine neue Verwendung von Omega-3-Fettsäure(n).

Aus der Fachliteratur ist es seit längerem bekannt, dass von Omega-3-Fettsäure(n), insbesondere hoch ungesättigten Omega-3-Fettsbure(n), vor allem EPA und DHA, entzündungshemmende und kardioprotektive Wirkungen ausgehen. Bei täglicher Einnahme von EPA und DHA in Dosen zwischen 0,5 und 1 g über mehrere Wochen lassen sich bereits positive Effekte erzielen. Nach einer Behandlung mit einem Gramm EPA und DHA pro Tag über sechs Monate konnte eine merkliche Verringerung des Herzinfarktrisikos festgestellt werden (G Ital Cardiol 1993; 23:1053-1061 j. Auch die Behandlung schmerzhafter Entzündungen und rheumatischer Erkrankungen mit (Omega-3-Fettsäure(n) zeigt nach mindestens einem Monat positive Wirkungen (Alair A.B., et. al. Nutrition (2005); 1, 2, 131-136). Ferner sind die Verwendung von Omega-3-Fettsäuren zur Langzeitbehandlung von Dermatitis und Psoriasis (J Am Acad Dematol. (1998); 38, 539-547) sowie zur Behandlung verschiedener prämenstrualer Beschwerden bekannt (Deutsch B. et. al. Nutrition Research (2000); 5 (20), 621-631 (11)).

Der Erfindung liegt die Aufgabe zugrunde, weitere Verwendungsmöglichkeiten für Omega-3-Fettsäure(n) zu erschließen.

Erfindungsgemäß werden gemäß Anspruch 1 Omega-3-Fettsäure(n) in einem Mittel zur vorbeugenden Bekämpfung von Muskelkaterschmerzen eingesetzt.

Der Erfinder hat erkannt, dass sich Omega-3-Fettsäuren über die bekannten, oben und in Anspruch 1 beschriebenen Wirkungen hinaus auch unmittelbar zur Linderung akuter Schmerzen eignen und insoweit auch eine kurzfristige, innerhalb von Stunden einsetzende Wirkung entfalten.

Bei der Schmerzbekämpfung besonders wirksam sind hochungesättigte Fettsäuren, vor allem EPA (Eicospentaensäure) und DHA (Docosahexansäure).

Eine kurzfristig einsetzende Wirkung als Schmerzmittel ergibt sich bei hoher Dosierung der Omega-3-Fettsäure(n), z.B. bei einer Dosierungsmenge von mehr als 2g, insbesondere von mehr als 3 bis 4g.

Vorzugsweise wird zur Schmerzbekämpfung die gesamte Dosierungsmenge einmalig zugeführt.

Der Wirkungsmechanismus der Schmerzlinderung hat Gemeinsamkeiten mit demjenigen des Aspirin-Wirkstoffs. Die Zufuhr von Omega-3-Fettsäure(n), insbesondere in hoher Dosierung, vermindert oder blockiert kompetitiv die Erzeugung entzündungsfördernder, Schmerzen bewirkender Substanzen, insbesondere Gewebshormonen, aus Omega-6-Fettsäure(n) über das COX-Enzym.

Gleichzeitig werden über das gleiche COX-Enzym aus den zugeführten Omega-3-Fettsäure(n) selbst entzündungshemmende Substanzen gebildet.

Vorzugsweise ist das die Omega-3-Fettsdure(n) enthaltende Mittel frei von anderen Fettsäuren, insbesondere Omega-6-Fettsäuren.

Das Mittel enthält als wirksame Substanzen ausschließlich Omega-3-Fettsäure(n) oder/und Verbindungen der Omega-3-Fettsäure(n). Als Zusätze kommen Geschmacksstoffe in Betracht.

Das die Omega-3-Fettsäure(n) enthaltende Mittel kann zur Schmerzbekämpfung z.B. bei Entzündungen und Verletzungen verwendet werden.

Unter anderem lässt sich das Mittel zur Bekämpfung von Kopfschmerzen, Zahnschmerzen, Gelenkschmerzen, Menstrualbeschwerden oder Muskelkater verwenden.

Vorteilhaft lässt sich das Mittel, insbesondere gegen Muskelkaterschmerzen, vor beugend einsetzen, z.B. nach längeren Trainingspausen vor der Wiederaufnahme des Trainings. Die vorbeugende Wirkung gegen Muskelkaterschmerzen beruht ferner auf einer Erhöhung der Elastizität der Zellen in den sog. Z-Scheiben und einer damit verbundenen Verminderung der Rissbildung. Die Einnahme von Omega-3-Fettsäure(n) kurz vor einer überdurchschnittlich starken Muskelbeanspruchung, z.B. vor einem Tennisturnier, verhindert oder mildert merklich die Entstehung von Muskelkater.

Die Wirkung des Mittels wird nachfolgend anhand mehrerer Beispiele beschrieben.

### 1. Beispiel (nicht beansprucht)

Die Wirkung gegen Schmerzen verschiedener Art, vor allem Kopfschmerzen, wurde an einer Vielzahl von Probanten getestet. Bei jeweils einmaliger Verabreichung von 3g hochungesättigter Omega-3-Fettsäure(n) (Produkt PUR3 der K.D. Pharma Bexbach GmbH: 60 % EPA + 20 % DHA + 10 % weitere Omega-3-Fettsäuren) wurde ein merkliches Nachlassen der Schmerzen innerhalb eines Tages, bei Kopfschmerzen sogar innerhalb von wenigen Stunden, erreicht.

### 2. Beispiel

Der Anmelder hat die Muskelkater vorbeugende Wirkung von Omega-3-Fettsäure(n) u.a. an sich selbst untersucht. Die jeweilige Einnahme von 3g Omega-3-Fettsäure(n) (Produkt K.D. Pharma Bexbach GmbH: 70 % EPA + 12 % DHA) einen Tag vor einer Muskelbeanspruchung (langandauernde gymnastische Übung, Bergwanderung, Gartenarbeit) verhinderte in allen beobachteten Fällen ganz oder teilweise das Auftreten von Muskelkater. Die Wirkung lässt nach, wenn die Einnahme frühzeitiger als drei Tage vor der Beanspruchung erfolgt.

### 3. Beispiel

Eine Menge von 4g Omega-3-Fettsäure(n) (K.D. Pharma - Produkt: 39 % EPA + 43 % DHA) wurde am Vormittag eines um 14.00 Uhr beginnenden Tennisturniers von zehn Personen eingenommen. Für alle diese Personen stellte das Turnier eine Muskelbeanspruchung dar, die nach Intensität und Dauer über das gewohnte Maß ihrer sportlichen Betätigung mehr oder weniger hinausging und erfahrungsgemäß zu Muskelkater geführt hätte. Bei keiner der betreffenden Person trat jedoch Muskelkater in nennenswertem Umfang auf.

## Patentansprüche

1. Verwendung EPA-enthaltender Omega-3-Fettsäure(n) für die Herstellung eines Mittels zur vorbeugenden Bekämpfung von Muskelkaterschmerzen durch Zuführung des Mittels vor einer Muskelbeanspruchung, wobei das Mittel als Wirksubstanz allein die Omega-3-Fettsäure(n) frei oder in gebundener Form enthält und zur vorbeugenden Bekämpfung der Muskelkaterschmerzen eine 3 bis 4 g der Omega-3-Fettsäure enthaltende Dosierungsmenge des Mittels frühestens drei Tage vor der Muskelbeanspruchung, oral zuzuführen ist.

2. Verwendung nach Anspruch 1,
**dadurch gekennzeichnet,**
**dass** die Omega-3-Fettsäure(n) neben EPA, die Omega-3-Fettsäure(n) DHA, DPA, ETA, 21:5n3 und/oder SDA umfassen.

3. Verwendung nach einem der Ansprüche 1 bis 2,
**dadurch gekennzeichnet,**
**dass** die Omega-3-Fettsäure(n) als Ester oder Salze verwendet werden.

4. Verwendung nach einem der Ansprüche 1 bis 3,
**dadurch gekennzeichnet,**
**dass** das Mittel frei von anderen Fettsäuren ist.

5. Verwendung nach Anspruch 4,
**dadurch gekennzeichnet,**
**dass** das Mittel neben der Omega-3-Fettsöure(n) als zur Vorbeugung alleiniger Wirksubstanz einen Geschmacksstoff enthält.

6. Verwendung nach einem der Ansprüche 1 bis 5,
**dadurch gekennzeichnet,**
**dass** die Omega-3-Fettsäure(n) in Form von Kapseln, Tabletten oder/und Flüssigkeiten verabreicht werden.

## Claims

1. Use of EPA-comprising omega-3 fatty acid(s) for producing a composition for the preventive control of pain from muscle soreness by supply of the composition before a muscular strain, the composition comprising as active substance solely the omega-3 fatty acid(s), free or in bound form, and the pain from muscle soreness being preventively controlled by the supply of the composition orally in a dosage amount comprising 3 to 4 g of the omega-3 fatty acid no earlier than three days before the muscular strain.

2. Use according to Claim 1, **characterized in that**, in addition to EPA, the omega-3 fatty acid(s) include the omega-3 fatty acid(s) DHA, DPA, ETA, 21:5n3 and/or SDA.

3. Use according to either of Claims 1 and 2, **characterized in that** the omega-3 fatty acid(s) are used as esters or salts.

4. Use according to any of Claims 1 to 3, **characterized in that** the composition is free of other fatty acids.

5. Use according to Claim 4, **characterized in that**, in addition to the omega-3 fatty acid(s) as the sole active substance for prevention, the composition comprises a flavouring.

6. Use according to any of Claims 1 to 5, **characterized in that** the omega-3 fatty acid (s) are administered in the form of capsules, tablets or/and liquids.

## Revendications

1. Utilisation d'acide(s) gras oméga-3 contenant du EPA pour la production d'un remède destiné à lutter de façon préventive contre les douleurs de courbatures musculaires par administration du remède avant une sollicitation musculaire, dans laquelle le remède contient à titre de substance active uniquement le/les acide(s) gras oméga-3 sous forme libre ou sous forme liée et, pour la lutte préventive des douleurs de courbatures musculaires, une quantité dosée du remède contenant 3 à 4 g des acides gras oméga-3 est administrée par voie orale au plus tôt trois jours avant l'activation musculaire.

2. Utilisation selon la revendication 1,
**caractérisée en ce que** le/les acide(s) gras oméga-3 contien(nen)t, outre du EPA, le/les acide(s) gras oméga-3 DHA, DPA, ETA, 21:son3 et/ou SDA.

3. Utilisation selon l'une des revendications 1 à 2,
**caractérisée en ce que** le/les acide(s) gras oméga-3 est/sont utilisé(s) sous forme d'esters ou de sels.

4. Utilisation selon l'une des revendications 1 à 3,
**caractérisée en ce que** le remède est exempt d'autres acides gras.

5. Utilisation selon la revendication 4,
**caractérisée en ce que** le remède contient en sus un agent de sapidité du/des acide(s) gras oméga-3 à titre de substance active unique pour la prévention.

6. Utilisation selon l'une des revendications 1 à 5,
**caractérisée en ce que** le/les acide(s) gras oméga-3 est/sont administré(s) sous la forme de capsules, de dragées et/ou de liquide.
